Europäisches Patentamt

European Patent Office

Office européen des brevets

(11) Publication number: 0 226 044 B1

# EUROPEAN PATENT SPECIFICATION

(45) Date of publication of patent specification: 12.08.92

(51) Int. Cl.5: A61B 8/06, G01P 5/00

(21) Application number: 86115755.0

(22) Date of filing: 13.11.86

(54) **Display of a stream line of an inhomogeneous flowing medium.**

(30) Priority: **14.11.85 JP 255401/85**

(43) Date of publication of application:
**24.06.87 Bulletin  87/26**

(45) Publication of the grant of the patent:
**12.08.92 Bulletin  92/33**

(84) Designated Contracting States:
**DE FR GB SE**

(56) References cited:
**EP-A- 0 008 517**
**EP-A- 0 135 170**
**EP-A- 0 177 942**

**PROCEEDINGS OF THE IEEE THIRD WORK-ING CONFERENCE ON CURRENT MEASURE-MENT, Virginia, 22nd-24th January 1986, pages 78-81, IEEE, US; S.F. CLIFFORD et al.: "Space-time analysis of acoustic scintilla-tions in ocean current sensing"**

(73) Proprietor: **FUJITSU LIMITED**
**1015, Kamikodanaka Nakahara-ku**
**Kawasaki-shi Kanagawa 211(JP)**

(72) Inventor: **Miwa, Hirohide Fujitsu Ltd. Pat. Dept.**
**Kosugi Fujitsu Building 1812-10**
**Shimonumabe**
**Nakahara-ku Kawasaki-shi Kanagawa 211(JP)**
Inventor: **Shimura, Takaki Fujitsu Ltd. Pat. Dept.**
**Kosugi Fujitsu Building 1812-10**
**Shimonumabe**
**Nakahara-ku Kawasaki-shi Kanagawa 211(JP)**
Inventor: **Yanashima, Tadahiko Fujitsu Ltd. Pat. Dept.**
**Kosugi Fujitsu Building 1812-10**
**Shimonumabe**
**Nakahara-ku Kawasaki-shi Kanagawa 211(JP)**
Inventor: **Amemiya, Shinichi Fujitsu Ltd. Pat. Dept.**
**Kosugi Fujitsu Building 1812-10**
**Shimonumabe**
**Nakahara-ku Kawasaki-shi Kanagawa 211(JP)**

(74) Representative: **Sunderland, James Harry et al**
**HASELTINE LAKE & CO Hazlitt House 28**
**Southampton Buildings Chancery Lane**
**London WC2A 1AT(GB)**

## Description

This invention relates to the display of stream lines of inhomogeneous flowing mediums.

In the art of medical electronics, it has been proposed to use an ultrasonic wave tomograph to display condition or motion of human organs on a cathode ray tube (CRT). Recently, it has been proposed to use a Doppler tomograph to obtain a display of blood motion, showing a "map" of blood flow on a two dimensional tomogram, using a colour display. For example, a bloodstream approaching an ultrasonic pulse transmitting and receiving transducer is coloured red and a stream going away from the transducer is coloured blue. The colour tone is varied in accordance with the speed of the bloodstream. An indication of the speed of the bloodstream is obtained from a frequency shift, phase shift or a distortion of pulse form present in an echo pulse and caused by the Doppler effect. A process whereby an indication of speed of a moving body is obtained from such data is called a Doppler process, and hence a tomograph using such a process is called a Doppler tomograph.

The following U.S. patents relate to Doppler tomography intended for displaying blood:-
4,182,173 by Papadofrangakis et al. Jan. 8, 1980;
4,476 874 by Taenzer et al. Oct. 16, 1984;
4,509,525 by Seo. Apr. 9, 1985.

In Doppler tomography the measured speed of a blood flow is the relative speed of the bloodstream concerned in the scanning direction of the ultrasonic beam used. Therefore, measured speed varies when the scanning direction is varied, and as a limit measured blood speed is zero when the bloodstream is scanned orthogonally. If the scanning direction is further rotated, the measured blood speed is reversed.

An attempt to overcome these problems has been proposed by K. Machii et al, in proceedings of WFUMB 1985, page 382, as "Clinical Usefulness of Power Mode Two Dimensional Doppler Colour Flow Mapping". In accordance with this proposal a speed component of blood included in a Doppler tomogram taken by power echo of an ultrasonic beam is detected. The application of this proposal is limited to blood speeds of less than 0.1 m/s. These are very slow speeds.

Moreover, the previously proposed ultrasonic tomograph cannot show blood stream-lines. Essentially, a blood stream-line is parallel to the relevant blood vessel. Therefore, the need to show blood stream-lines has not in general been great. However, it is important to accumulate information concerning blood flow in the heart. The heart is a chamber rather than a tube so the flow of blood in a ventricle cannot be anticipated from its appearance. Accordingly it is important to display real speed and direction of a stream line of blood flow in a tomogram. Previously, however, so far as the inventors are aware, no means have been provided which can show stream lines of blood flow in the human heart.

According to the present invention there is provided a method of detecting a stream line of a flow of an inhomogeneous medium, and displaying a representation of the stream line in real time, comprising:-

(a) sending out ultrasonic signals to the object within which the flow occurs, to scan the object along a plurality of scanning lines, covering a scanning field, so that points within the object are scanned a plurality of times;

(b) detecting echoes of the ultrasonic signals from the object;

(c) forming an image representing differences between echoes received from the same point within the object but arising from ultrasonic signals sent out at different times, thereby to detect and display the locus of movement of features which move with the flow, as a representation of the stream line.

According to the present invention there is also provided a device for detecting a stream line of a flow of an inhomogeneous medium, and displaying a representation of the stream line in real time, by scanning the object in which the flow occurs with ultrasonic signals and processing echoes of those signals, the device comprising:-

ultrasonic transducer means operable to send out and to receive the said ultrasonic signals and their echoes;

transmission circuitry operable to excite the transducer means to cause the ultrasonic transducer means to send out the said ultrasonic signals so that points in the object are scanned a plurality of times;

receiving circuitry operable to receive electrical signals provided by the ultrasonic transducer means in response to echoes of the said ultrasonic signals from the object;

an echo signal generator operable to process electrical signals from the receiving circuitry to provide echo signals in a digital form suitable for image processing;

a plurality of sub-frame memory means operable to store echo signals in a plurality of memory planes in accordance with address signals;

a stream line processor (SLP), operable to process data stored in the sub-frame memory means to obtain data representing segments of stream lines, comprising:-

a digital scan converter which provides an input echo signal with an address signal for use in storage in the sub-frame memory means;

a digital signal processor which takes differences between echo signals relating to the same point in the object to obtain data representing images of speckles in the flow and takes differences representing speckle movement in the flow; and

a plurality of switches for controlling flow of data and address signals between the digital scan converter, the digital signal processor and the sub-frame memory means;

an image memory means for storing data relating to segments of stream lines; and

a display circuit operable on the basis of data stored in the image memory means and to generate image signals for representing segments of the stream line on a CRT (cathode ray tube) display.

An embodiment of the present invention can provide a method of displaying a stream line of an inhomogenous flowing medium.

An embodiment of the present invention can provide a device for showing a stream line of an inhomogenous flowing medium.

An embodiment of the present invention can provide a tomograph which can show a stream line of blood flow in the human heart.

Embodiments of the present invention can provide for real-time display of a blood flow stream line.

An embodiment of the present invention observes, in effect, motion of speckles which appear in a tomogram of an inhomogeneous flow such as a bloodstream. For example, two tomograms, taken at timings separated by a very short time interval, during which interval correlation between speckles is retained, are used. From an image constructed from difference between the two tomograms, direction of motion and speed of the speckles are obtained. By a proper graphic process, segments of stream lines are shown in the tomogram.

Taking two tomograms at timings separated by a very short time interval means that there may be insufficient time available to scan the entire extent of a desired scanning field. In this case, the scanning field, in an embodiment of the invention, is divided into m sub-fields, and in respect of each sub-field processing as mentioned above is performed. When processing in respect of a first sub-field is completed, the next sub-field is processed, and in such a manner the entire scanning field is composed.

According to the speed of flow to be measured and the required accuracy of measurement, several methods of field separation and scanning are provided in embodiments of the present invention.

Reference is made, by way of example, to the accompanying drawings, in which:-

Fig. 1 schematically illustrates imaging of speckles, wherein

Fig. 1(a) schematically illustrates an example of speckle motion, indicated by the superposition of two tomograms taken at times separated by a short time interval:

Fig. 1(b) schematically illustrates an image of speckles obtained from a difference between the two tomograms used in Fig. 1(a);

Fig. 1(c) schematically illustrates an image obtained from that Fig. 1(b), using absolute value processing;

Fig. 1(d) is an image corresponding to that of Fig. 1(b), but for a case in which three tomograms, taken successively at times separated by short time intervals, are employed;

Fig. 1(e) is an image corresponding to that of Fig. 1(c) but obtained by processing from the image of Fig. 1(d); and

Fig. 1(f) is an image obtained by a thinning process applied to the image of Fig. 1(e), showing segments of stream lines;

Fig. 2 illustrates a scanning method employed in a first embodiment of the present invention, wherein

Fig. 2(a) illustrates division of a scanning field, and

Fig. 2(b) is a graphical illustration of time variation of scan angle;

Fig. 3 is a graphical illustration of time variation of scan angles in a scanning method employed in a second embodiment of the present invention which is applicable to a more precise measurement;

Fig. 4 illustrates a scanning method of a third embodiment of the present invention, which can reduce noise caused by motion of a target or which is applicable for measuring a very fast flow; in Fig. 4 small black dots indicate ultrasonic pulse shots at respective scan angles;

Fig. 5 is a schematic block diagram illustrating circuit constitution of an ultrasonic tomograph in accordance with an embodiment of the present invention, and

Fig. 6 is a schematic block diagram illustrating a stream line processor and sub-frame memories of the tomograph of Fig. 5, illustrating their operations in the circuit of Fig. 5.

First, a tomogram image derived from signals originating from ultrasonic pulses reflected from an inhomogeneous flowing medium, such as blood, will be discussed.

Blood is composed of a mixture of red blood corpuscles, hemoleukocytes and blood platelets immersed in blood serum. Mean density of blood is almost uniform in the heart, for example. However, the microscopic spatial arrangement of blood components, red blood corpuscles for example, is random. A reflected ultrasonic wave is composed of waves reflected or scattered from each of those various red blood corpuscles respectively. Since these reflected waves interfere with each other, an image obtained from reflected waves exhibits speckles consisting of randomly arranged white (for example) portions (indicating strong reflections) and dark (for example) portions (indicating weak reflections). Usually, such a white portion is called a "speckle".

Reflections of ultrasonic waves from blood are extremely weak as compared with those from muscles, the heart wall or heart membranes. So, reflections from blood cannot be observed in ordinary B mode image display, in which reflected wave intensity is represented by image brightness on a CRT. However, as will be described later, if two tomograms are taken in sequence, and an image corresponding to differences between these two tomograms is extracted, the strong image of muscles and heart membranes, etc., which are not moving, disappear. Only signals from portions that have moved during the interval between the tomograms are left on the display. Such a "difference" image can provide an image of blood stream flow, and hence speckles appear.

The size of a speckle in the observation of blood flow in this way varies depending upon aperture of the ultrasonic transducer, focusing of the ultrasonic beam employed, depth of the reflecting body (blood) (distance from transducer), frequency of the ultrasonic waves employed, and bandwidth of the ultrasonic receiver, and so on. In the following analysis, the shape of a speckle is approximated to a circle having a diameter of 5 mm, and the depth of measurement is assumed to be 10 cm.

Next, the formation of segments of stream lines will be described.

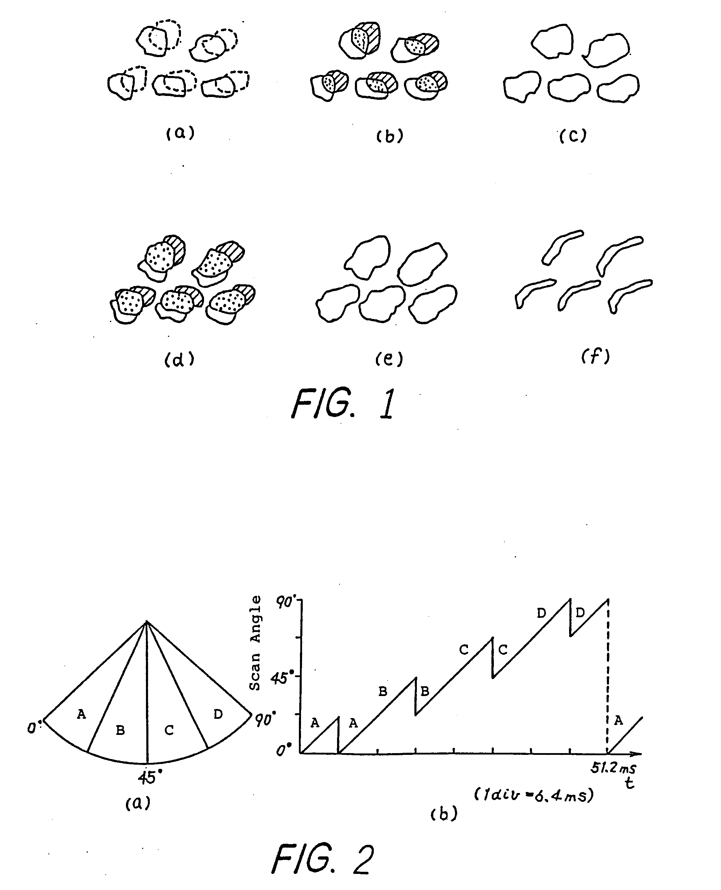

Fig. 1(a) is an image of speckles. In the Figure, two images of flowing speckles measured or taken sequentially, with a short time interval between them, are superposed. The broken lines indicate speckles observed in the first measurement, and the solid lines indicate the same speckles observed in the second measurement. It is necessary that the time interval $\Delta T$ between the two measurements be small, so that the image of respective speckles obtained in the two measurements are superposed on (at least partially overlap) one another to retain spatial correlation. If $\Delta T$ is too large, the first (former) images of the speckles separate for the second (latter) images, and since the shape of a speckle varies rapidly, correlation between the speckles is lost, and it is difficult to distinguish which speckle has moved to where. $\Delta T$ should be varied depending on the speed of motion of the speckles to be measured.

When the images of Fig. 1(a) are shown with brightness modulation, that is, as monochromatic images, and when an image showing differences of brightness between the first and second images is composed, it is schematically illustrated in Fig. 1-(b). In the Figure, the dotted portions correspond to regions of small brightness difference (between the first and second images) indicating parts where speckles overlap; the white portions indicate regions of brightness of difference of a positive value (e.g. first image brighter than second in these regions), and the hatched portions indicate regions of brightness of difference of a negative value (e.g. first image darker than second). If the images are colour-modulated and positive difference value portions (white portions) coloured red and negative difference value portions (hatched portions) are coloured green, the central portions (dotted portions) become a mixture of red and green, so it is possible to determine that the speckles have moved in a direction from green to red. The distance between the red and green portions corresponds to the speed of the speckles. Thus it is possible to image the direction and speed of a bloodstream by interpretation of the speckles.

As has been mentioned above, if $\Delta T$ is too large, correlation between first and second measurements is lost, and it is impossible to judge the direction of motion of the speckles. On the other hand, if $\Delta T$ is too small, the variation or displacement of speckles is too small to enable movement to be indicated.

If only absolute brightness values are used to indicate differences between the first and second images of Fig. 1(a) the resultant image on the CRT becomes as schematically indicated in Fig. 1(c). At the two ends of the speckles, the brightness is high, and at the centre portion, the brightness is small. This picture resembles a photograph of moving particles taken with a properly long exposure. The dislocation or displacement of the images indicates the direction of motion, and the length of the dislocation or displacement indicates the speed of motion.

It is possible to repeat the above process several times. An example of this is schematically illustrated in Figs. 1(d) and (e). The Figures show images obtained with the processes of Fig. 1(b) and Fig. 1(c) carried out twice in each case. Namely the repetition number q is 2. In this example, three tomograms have been taken. Using the first and second, and second and third tomograms, the above-mentioned processes are performed twice, and the obtained images superposed on each other. Fig. 1(d) corresponds to Fig. 1(b), the white portions may be represented on a colour display as red, the hatched portions as green, and the dotted portions become a yellowish mixture of red and green. Fig. 1(e) corresponds to Fig. 1(c). Fig. 1(d) and (e) have advantage that the images provided have greater resemblance to a stream line.

Generally, the images of speckles are brightest at their centres, and become darker towards their peripheries. Therefore, the formed segments of stream lines indicated in Fig. 1(d) and (e) are brighter at their centre portions, and darker on both sides of the images. Therefore, if brightness is digitized using an appropriate threshold value (namely if the portions brighter than the threshold value are emphasized to maximum brightness (100% bright), and portions of brightness less than the threshold value are cut down to zero brightness) the images of Fig. 1(e) are thinned, as illustrated in Fig. 1(f). Compared to the broad features of Figs. 1(d) and (e), the images of Fig. 1(f) resemble more nearly segments of stream lines.

As has been mentioned above, it is necessary to retain the spatial correlation of speckles. When the speckle images of two tomograms are superposed as shown in Fig. 1(a), if a speckle observed in the first measurement (broken lines) contacts another speckle image, it will cause a misjudgment of the stream line or speed of flow. In practice, diameter of a speckle is almost equal to the spacing between speckles. So the displacement of a speckle between successive measurements must be less than the diameter of the speckle. This limits the maximum length of the segment of a stream line to be displayed on a tomogram. Usually speckle diameter is almost equal to the spacing between speckles. So the maximum length of a stream line segment is twice the speckle diameter. For example, if speckle diameter is 5 mm, the length of a stream line segment which can be shown on the tomogram is less than 10 mm.

The length of stream line segments can be varied by varying the diameter of the speckles. The diameter of the speckles may be varied by varying the aperture of the transducer, focusing of the beam and wave length of the ultrasonic wave, etc. However, most effective for obtaining a long stream line is the introduction of some kind of echo enhancing particle, such as bubbles, into the blood stream. Compared to measurement using speckles, such an introduced particle (bubble for example) does not change its shape during motion, and the size of and spacing between particles may be controlled at will. The movement of a particle (e.g. bubble) may be traced, without losing its spatial correlation, for a relatively long time. So, with such particles, the repetition number q of the measurement may be increased to provide a long stream line as compared with that shown in Fig. 1(f). The effect is similar to that which would be achieved if the motion of a particle were traced by a motion picture, and then the frames of the motion picture were superposed on each other.

The conditions that $\Delta T$, the measurement time interval, should satisfy will be considered. Let the expected maximum speed of the bloodstream be V mm/ms, and let speckle size correspond to a diameter D mm. In order to form an image of a stream line, it is necessary for there to be at least an overlapped portion between the images of the same speckle when first and second tomograms are superposed to each other. Therefore, it is necessary that

$$V \cdot \Delta T < D. \qquad (1)$$

If speckle size is assumed to be equal to the spacing between speckles, inequality (1) becomes also the condition that one speckle does not overlap other speckles. This also determines the maximum length of stream line segments.

If measurement is repeated q times (if $q+1$ tomograms are used) to obtain a more precise indication of a stream line segment, the condition becomes

$$V \cdot \Delta T \cdot q < D. \qquad (2)$$

On the other hand, if artificial echo enhancing particles such as bubbles are introduced, as has been described above, it is possible to control the mean spacing between the bubbles to be large compared to the size of the bubbles, and in this case conditions (1) and (2) become unnecessary.

Embodiments of the present invention will be described below as they relate to the provision of tomographs for displaying bloodstream in the human heart. For such purpose, it is necessary to provide a measuring depth of at least 150 mm. Since sound velocity in the human body is about 1.5 mm/$\mu$s, it takes 0.2 ms for an ultrasonic wave to travel to and return from a depth of 150 mm. It is desirable to provide a frame rate of 15-30 frames/s in order to display the bloodstream of a beating heart. It is said that the speed of an abnormal bloodstream which flows backward from a gap of an incomplete cardiac valve is sometimes as high as 2.5 m/s. However, the embodiment of the present invention described below is designed to display bloodstream speeds of up to 1 m/s. It is desirable to scan the object (the heart) with a fan shaped scanning field having a scan angle of 45-90°, and to display the scanning field as a frame on a CRT. It is desirable to provide 64-128 scanning lines per 90° for ordinary B mode display.

Fig. 2 illustrates an ultrasonic beam scanning method employed in a first embodiment of the present invention. As illustrated in Fig. 2(a), a scanning field of 90° is divided into four sub-fields A, B, C and D, each having a scan angle of 22.5°. Fig. 2(b) illustrates the relationship between scan angle and scan time, over the time required to scan an entire frame of the scanning field. The scanning line density is 128 lines/90°. So, each sub-field is scanned by 32 scanning lines respectively. As indicated above, it takes 0.2 ms to obtain each scanning line, and it takes 6.4 (0.2 × 32) ms to scan over one sub-field. Each sub-field is scanned twice and, therefore, measurement in relation to one sub-field takes 12.8 ms. When a measurement in relation to sub-field A is finished, sub-field B is scanned, in succession. It thus requires 51.2 ms to complete measurement in respect of the entire scanning field of 90°. So, the frame rate is 20 frames/s, and the time interval between scannings ΔT is 6.4 ms. These measuring constants almost satisfy inequality (1).

Data obtained from ultrasonic echo signals is stored successively in a memory, and after measurement in respect of one sub-field is completed, image processing is effected to obtain segments of stream lines as described above. The time needed for such processing is very short compared to the time needed for measurement in respect of one sub-field. So, such processing in relation to one sub-field is completed while measurement in respect of a next sub-field is going on, and the results are displayed on a CRT as a sub-frame. Images of sub-fields obtained in such manner are displayed successively on the CRT to construct an entire frame of the scanning field.

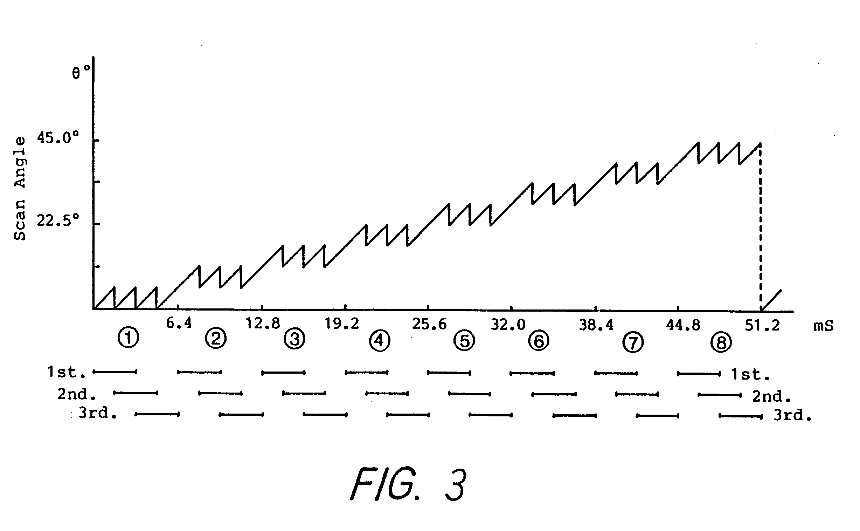

A scanning method employed in a second embodiment of the present invention is illustrated in Fig. 3. This embodiment can provide for a more precise measurement of stream lines.

The entire scanning field of 45° is divided into 8 sub-fields, and measurements are repeated three times for each of the sub-fields; therefore, the repetition number q is 3. The scanning line density is 128 lines/90°. So, the number of scanning lines p which covers one sub-field is 8, and the time interval between measurements, ΔT, is 1.6 ms (0.2 × 8). So more precise stream line determination can be available.

Fig. 3 indicates time variation of the scan angle of the supersonic (ultrasonic) beam. In the Figure, the ordinate corresponds to scan angle and the abscissa to time in ms. Circled numerals along the abscissa indicate the relevant sub-fields being scanned at the indicated times and scan angles. As shown in the Figure, each sub-field is scanned four times. Small horizontal bars beneath the circled numerals indicate pairs of scannings, from which scan data is processed to obtain differences. For each sub-field, the first processing is effected using the difference between first and second scanning data indicated by the first bar. The second processing is effected using the second and the third scanning data indicated by the second bar, and the third processing is effected using the third and fourth scanning data indicated by the third bar. For each processing ΔT is 1.6 ms. Images of moving speckles are obtained respectively from these three processings and stream lines are obtained by superposing these images. On the CRT, sub-frames are composed in each 6.4 ms, and the total time to complete a frame is 51.2 ms, so the frame rate becomes approximately 20 frames/s.

In the second embodiment, as compared with the first embodiment, ΔT, the time difference or interval between two images to be processed is reduced to one-fourth. Therefore, spatial wavelength of noise induced by slow motion of organs arising from breathing or slippage of the transducer during the measurements is reduced to one-fourth. So, it becomes easy to apply a filter to remove such noise, and hence a better image is obtained.

A third embodiment of the present invention employs a scanning method intended to suppress such noise to as low a level as possible. The time interval to take a difference is reduced to 0.2 ms, namely, the difference is taken between successive shots of the supersonic (ultrasonic) beam. The scanning field is 45°, it is divided into 8 sub-fields (m = 8), and each sub-field is scanned by 8 scanning lines respectively (p = 8). Each scanning line is scanned twice (in succession) repeatedly, and echo information is obtained from the difference between these two shots. Therefore noise caused by the motion of the target is reduced to minimum, and only the fast movement of blood flow is left. Such double scanning is repeated three times (q = 3) in relation to each sub-field.

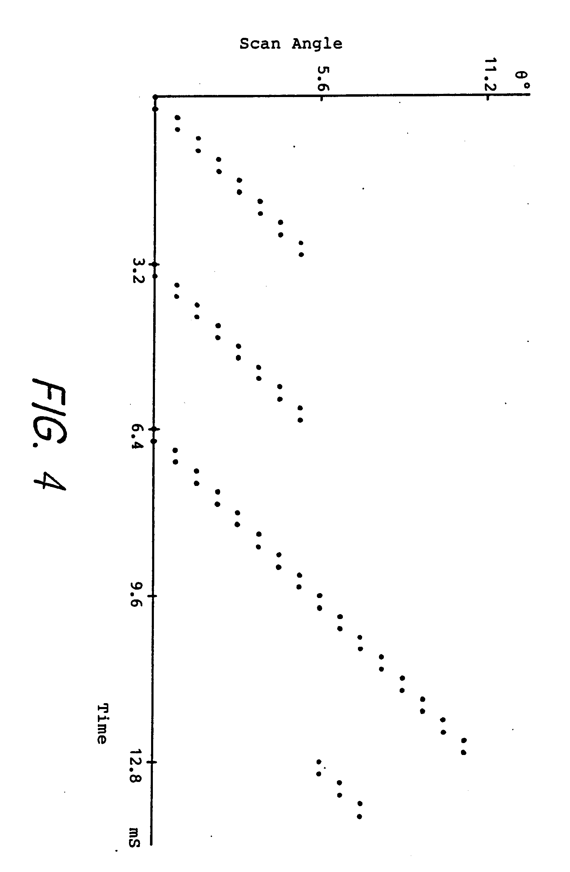

Fig. 4 illustrates the time variation of beam scan angle for the method employed in the third embodiment. Abscissa is time in ms, and ordinate is scan angle. Black dots in Fig. 4 indicate ultrasonic beam shots; therefore each spot corresponds to one line scanning, and from each two spots aligned horizontally in Fig. 4 (i.e. from the scannings of the same line), information relating to one scanning line is obtained. Though Fig. 4 illustrates only a part of a total scanning cycle, it will be clear that the scan angle is increased stepwise after every second shot. Each sub-field is scanned with eight double scans (it takes 3.2 ms), and such scanning is repeated three times for each sub-field. When the process for one sub-field is completed (it takes 9.6 ms), the image is displayed on a CRT, and then the next sub-field is processed successively. The time required for measurement in respect of one frame is 76.8 ms, so the frame rate becomes 13 frames/sec.

In practice, there can often occur a requirement for images such that the total scan angle is 90°, the frame rate is 30 frames/sec., and the scanning line density is 128 lines/90°. The above-described embodiments of the present invention do not satisfy these requirements. However, in the observation of the heart, for example, phenomena are repeated in synchrony with the beating of the heart. Accordingly, it is possible to apply stroboscopic technology. For example, it is possible to provide an image of blood flow at a predetermined time phase with respect to the R wave of an electro-cardiogram, using measurements obtained in respect of several beats of the heart.

For example, using the scanning method of the above-described second or third embodiment of the present invention, if the total scan angle is 22.5°, m = 4, p = 8 and q = 3, the scanning satisfies the above requirements in terms of scanning line density and frame rate. Therefore, by adding four images each having a 22.5° frame angle but having directions differing from one another but 22.5°, the images taken from or in respect of four successive cardiac pulses, it is possible to synthesize a 90° frame image. Such stroboscopic synthesizing is known in the art so further description is omitted.

In the description of embodiments of the present invention given above, the original signals from which images are formed are assumed to be simply echo signals. However, in the art of ultrasonic imaging, various types of input signal are known. For example, a time gain controlled signal, a logarithmic signal, an orthogonal detected signal, a power signal and so on. Embodiments of the present invention can employ such signals as input.

It will be clear that the above-described embodiments of the present invention employ scanning methods to accumulate information on the reflection of ultrasonic waves. Various modifications are possible; for example, it is possible to scan several times on the same scanning line to reduce noise and, after reduction of noise, use the (resultant) signal as the scanning line signal. The above-described embodiments are, therefore, to be considered in all respects as illustrative and not restrictive.

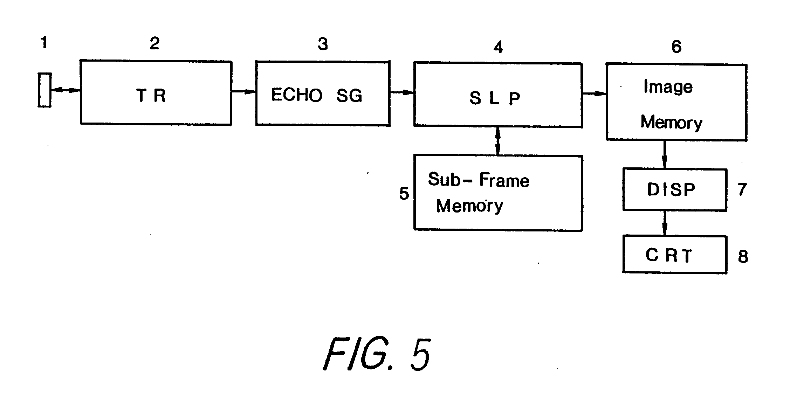

The configuration of a tomograph in accordance with an embodiment of the present invention is schematically illustrated in Fig. 5. 1 is a transducer which emits and receives an ultrasonic beam, 2 is a circuit for handling transmission or reception of ultrasonic waves (abbreviated to TR). Transducer 1 and circuit 2 are similar to such items used for an ordinary ultrasonic imaging device having a B mode display. 3 is an echo signal generator (ESG) which processes received echo signals and generates a signal having various kinds of characteristics preferable to obtain a desired image, for example, a logarithmic wave, a gain controlled echo signal, a power signal and so on. As has been indicated above, the use of any such processed signal is possible. Such signals are all well-known in the art, so further description is omitted.

The signal processed in the echo signal generator is mainly an analog signal, so the output signal of the echo signal generator is converted to a digital signal by a built-in analog to digital converter (A/D converter), and supplied to a stream line processor (SLP) 4. The stream line processor stores echo signals in a plurality of sub-frame memories 5, and reads out the memories to process the stored data as has been described above, to provide an image signal. Image signals are stored in an image memory 6. A display circuit (DISP) 7 reads out the image memory, and displays images on a CRT 8. Though it is not shown in the Figure, all of these processes and timing are controlled by a central processor unit (CPU).

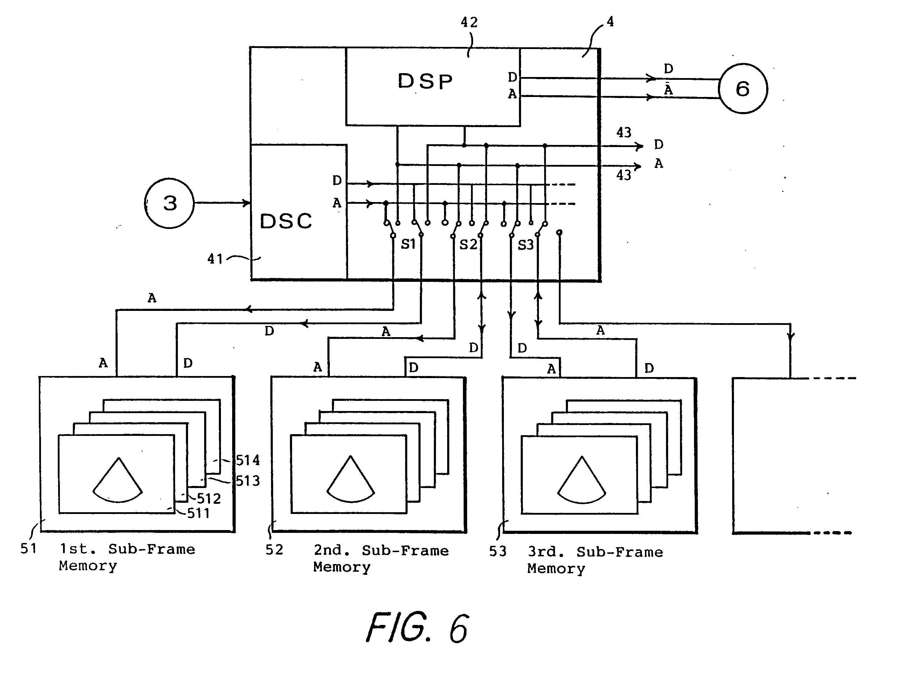

Operation in the stream line processor (SLP) 4 will be described briefly with reference to Fig. 6. The echo signal processed in the echo signal generator 3 is digitized and supplied to the SLP 4. The SLP comprises a digital scan converter (DSC) 41, which provides the input signal with an address for storage in sub-frame memory 5. The sub-frame memory 5 is composed of plurality of memory means which may be called, respectively, first, second, and third sub-frame memories and so on (51, 52, 53 ...), corresponding to the number of sub-frames m into which the scanning field is divided. The SLP 4 also comprises switches S1, S2, S3 and so on, respectively corresponding to the sub-frame memories. They are used to switch the flow of address and echo signals to the sub-frame memories or to a digital signal processor (DSP) 42. The I/O (input and output) ports of these circuitries and data bus lines connecting them are identified by notations A and D respectively. The directions of streams of data in the data buses are indicated by arrows.

For example, when a beam is scanning a first sub-field, the switch S1 is switched to the DSC side, and the other switches are switched to the DSP side, as shown in Fig. 6, so that echo data is stored in the first sub-frame memory 51. In the sub-field memories, for example in the first sub-field memory 51, the data is stored in memory planes 511, 512, 513 and so on, each plane corresponding to a first, second, third scanning, and so on. Such a memory device, its control and operation are well-known in the art, so further description is omitted for the sake of simplicity. During the time, when echo data is being stored in sub-frame memory 51, the other switches S2 to S4 are switched to the DSP side, so the DSP can carry out processing to obtain stream lines, using data stored in the second and third sub-frame memories and so on. The data processed by the DSP is sent to the image memory 6 and stored there.

When scanning in respect of the first sub-frame is completed, the switches S1 and S2 are switched respectively to the DSP and DSC sides. Echo data is then stored in the second sub-field memory 52, and data stored in the first sub-field memory 51 is processed by DSP 42. In such manner, the entire field is scanned and processed successively. The digital signal processor (DSP) 42 may be of any kind which can perform the required processing, as has been mentioned before.

Processes for display of an image such as is stored in the image memory 6 on a CRT 8 using a display circuit DISP 7 are known in the art, so description will be omitted. Usually, however, it is necessary to display in superposition streamline segments and an image of the target (heart for example). For such purpose, data obtained from the data buses 43, 43′ is applicable. This data includes image information of the target. Mixing this data with data stored in the image memory can be achieved using well-known processes.

As has been described, it is possible with an embodiment of the invention to provide for display in real time of segments of a stream line of a bloodstream in the heart. Although the description given above has been given with respect to blood flow, it will be clear that the invention may be applied to any other inhomogeneous flow.

Embodiments of the present invention provide methods and devices enabling real time display of a stream line of an inhomogeneous flowing medium, for example of blood flowing in the heart. The object (containing the flow of an inhomogeneous medium) is scanned several times using ultrasonic beam pulses. By taking differences between echoes appearing with a predetermined time interval at each point of the object, an image of speckles formed by the flow is formed. Such a process is repeated several times, to obtain the motion of the speckles. By taking a difference between the images of spatially correlated speckles taken with the time interval, the segments of the stream line are obtained. There are various ways of taking a difference between successive frames, successive lines, etc. According to such methods, several methods of scanning are disclosed to take the difference between the images.

An embodiment of the present invention provides a method to display in real time a stream line of an inhomogeneous flowing medium by scanning an object with ultrasonic wave beam pulses and processing the echoes thereof reflected from the object, comprising successive processes of:-

A) sending out pulses of the ultrasonic wave beam to scan the object so as to each point of the object is scanned plural times with a predetermined time interval;

B) forming images of speckles formed by the flow of said inhomogeneous flowing medium by taking a difference between two echoes appearing with said time interval at each point of a scanning line; and

C) obtaining locus of movement of said speckles having spatial correlation between each other during said time interval by taking a difference of the images of the speckles obtained by repetition of said process B.

An embodiment of the present invention provides a device to display in real time a stream line of an inhomogeneous flowing medium by scanning an object with ultrasonic wave beam pulses and processing the echoes thereof reflected from the object, comprising:

a transducer for converting electrical energy to sound energy or vise versa for sending out and receiving ultrasonic wave;

a transmitting circuit for exciting the transducer and make it generate a pulse of ultrasonic wave beam in a direction to scan the object so as to each point of the object is scanned plural times with a predetermined time interval;

a receiver which receives a signal from the transducer which converts the echo of ultrasonic wave from an object into an electrical signal;

an echo signal generator for processing the electrical signal of said receiver to provide an echo signal preferable for the image processing and converting it to a digital signal;

a plurality of sub-frame memory means for storing the echo signal;

a stream line processor (SLP) for processing the data stored in said sub-frame memory means to obtain the data of segments of stream lines;

an image memory means for storing the data of segments of stream lines; and

a display circuit for taking out the data stored in said image memory means and generating an image signal of the segments of stream line to display on a CRT (cathode ray tube).

## Claims

1. A method of detecting a stream line of a flow of an inhomogeneous medium flowing within an object, and displaying a representation of the stream line in real time, comprising:-

(a) sending out ultrasonic signals to the object within which the flow occurs, to scan the object along a plurality of scanning lines, covering a scanning field, so that points within the object are scanned a plurality of times;

(b) detecting echoes of the ultrasonic signals from the object;

(c) forming an image representing differences between echoes received from the same point within the object but arising from ultrasonic signals sent out at different times, thereby to detect and display the locus of movement of features which move with the flow, as a representation of the stream line.

2. A method as claimed in claim 1, wherein the said features are speckles.

3. A method as claimed in claim 1, wherein the said features are bubbles or particles introduced into the flow.

4. A method as claimed in claim 1, 2 or 3, wherein steps (a) to (c) are carried out in respect of each of a plurality of different scanning fields forming respective sub-fields of an overall composite scanning field.

5. A method as claimed in any preceding claim, wherein the or each step (a) comprises completing at least two scanning frames of the scanning field, and wherein step (c) comprises taking differences between echoes relating to the same scanning line but arising from ultrasonic signals sent out in different scanning frames.

6. A method as claimed in any preceding claim, wherein the or each step (a) comprises scanning each line of the scanning field at least twice in a scanning frame, and wherein step (c) comprises taking the differences between echoes relating to different scannings of the same scanning line in the same scanning frame.

7. A method as claimed in claim 2, or any one of claims 4 to 6 read as appended to claim 2, wherein the time interval $\Delta T$ between first and last times at which ultrasonic signals are sent out, echoes arising from which contribute to provision of the imaging of the same point in the object is less titan D/V where D is mean speckle diameter and V a maximum (anticipated) flow speed.

8. A method as claimed in any preceding claim, wherein the medium is blood and the object is the heart.

9. A method as claimed in claim 8, wherein steps (a) to (c) are repeated in synchrony with successive beats of the heart.

10. A method as claimed in any preceding claim, wherein the differences are compared with a threshold value, and quantized to a predetermined maximum or a predetermined minimum depending on whether or not they exceed the threshold value.

11. A device for detecting a stream line of a flow of an inhomogeneous medium flowing within an object, and displaying a representation of the stream line in real time, by scanning the object in which the flow occurs with ultrasonic signals and processing echoes of those signals, the device comprising:-

ultrasonic transducer means operable to send out and to receive the said ultrasonic signals and their echoes;

transmission circuitry operable to excite the transducer means to cause the ultrasonic transducer means to send out the said ultrasonic signals so that points in the object are scanned a plurality of times;

receiving circuitry operable to receive electrical signals provided by the ultrasonic transducer means in response to echoes of the said ultrasonic signals from the object;

an echo signal generator operable to process electrical signals from the receiving circuitry to provide echo signals in a digital form suitable for image processing;

a plurality of sub-frame memory means operable to store echo signals in a plurality of memory planes in accordance with address signals;

a stream line processor (SLP), operable to process data stored in the sub-frame memory means to obtain data representing segments of stream lines, comprising:-

a digital scan converter which provides an input echo signal with an address signal for use in storage in the sub-frame memory means;

a digital signal processor which takes differences between echo signals relating to the same point in the object to obtain data representing images of speckles in the flow and takes differences representing speckle movement in the flow; and

a plurality of switches for controlling flow of data and address signals between the digital scan converter, the digital signal processor and the sub-frame memory means;

an image memory means for storing data relating to segments of stream lines; and

a display circuit operable on the basis of data stored in the image memory means and to generate image signals for representing segments of the stream line on a cathode ray tube (CRT) display.

12. A device as claimed in claim 11, wherein the medium is blood and the time interval $\Delta T$ between first and last echo signals relating to the same point in the object, and between which a difference is taken, is less than D/V, where D is mean speckle diameter and V an anticipated maximum flow speed.

13. A device as claimed in any one of claims 11 or 12, wherein the digital signal processor (DSP) is operable to compare the value of a difference representing speckle movement in the flow with a threshold value, and to quantize the difference to a predetermined maximum or minimum value.

**Revendications**

1. Procédé de détection d'une ligne de flux dans un flot d'un milieu inhomogène circulant à l'intérieur d'un objet, et de visualiser une représentation de la ligne 40 flux en temps réel, comprenant :

    (a) l'émission de signaux ultrasonores vers l'objet à l'intérieur duquel le flot survient, pour balayer l'objet le long d'une pluralité de lignes de balayage, en recouvrant un champ de balayage, de telle sorte que les points à l'intérieur de l'objet soient balayés une pluralité de fois ;

    (b) la détection des échos de signaux ultrasonores provenant de l'objet ;

    (c) la formation d'une image représentant la différence entre les échos reçus d'un même point dans l'objet, mais arrivant de signaux ultrasonores émis à des instants différents, de façon à détecter et à visualiser le lieu du mouvement de caractéristiques qui se déplacent avec le flot, telle qu'une représentation de la ligne de flux.

2. Procédé selon la revendication 1, dans lequel lesdites caractéristiques sont des taches.

3. Procédé selon la revendication 1, dans lequel lesdites caractéristiques sont des bulles ou des particules introduites dans le flot.

4. Procédé selon les revendications 1, 2 ou 3 dans lequel les étapes (a) à (c) sont exécutées relativement à chacun d'une pluralité de champs de balayage différents formant des sous-champs respectifs d'un champ de balayage composite d'ensemble.

5. Procédé selon l'une quelconque des revendications précédentes, dans lequel chaque étape (a) comporte l'achèvement d'au moins deux trames de balayage dans le champ de balayage et dans lequel l'étape (c) comporte la prise de différences entre les échos concernant la même ligne de balayage mais arrivant de signaux ultrasonores émis à des trames de balayage différentes.

6. Procédé selon l'une quelconque des revendications précédentes, dans lequel chaque étape (a) comporte le balayage de chaque ligne du champ de balayage au moins deux fois dans une trame de balayage, et dans lequel l'étape (c) comporte la prise de différences entre les échos concernant divers balayage de la même ligne de balayage dans la même trame de balayage.

7. Procédé selon la revendication 2, ou selon l'une quelconque des revendications 4 à 6, liées à la revendication 2, dans lequel l'intervalle de temps $\Delta T$ entre la première et la dernière fois auxquelles les signaux ultrasonores ont été émis, dont les échos proviennent et qui contribuent à produire l'imagerie du même point de l'objet, est plus petit que D/V où D est le diamètre moyen de la tache et V la vitesse d'écoulement maximum envisagée.

8. Procédé selon l'une quelconque des revendications précédentes, dans lequel le milieu est du sang et l'objet est le coeur.

9. Procédé selon la revendication 8, dans lequel les étapes (a) à (c) sont répétées en synchronisme avec des battements successifs du coeur.

10. Procédé selon l'une quelconque des revendications précédentes, dans lequel les différences sont comparées avec une valeur de seuil, et numérisées sur un maximum prédéterminé ou un minimum prédéterminé dépendant du fait que elle excédent ou non la valeur de seuil.

11. Dispositif de détection d'une ligne de flux du flot d'un milieu inhomogène, circulant à l'intérieur d'un objet, et d'affichage d'une représentation de la ligne de flux en temps réel, en balayant l'objet des lequel le flot survient par des signaux ultrasonores et de traitement des échos de ces signaux, le dispositif comprenant :

    - des moyens transducteurs ultrasonores pouvant fonctionner de façon à émettre et à recevoir lesdits signaux ultrasonores ;

    - une circuiterie de transmission susceptible d'exciter les moyens transducteurs pour provoquer l'émission par les moyens transducteurs ultrasonores des dits signaux ultrasonores de telle sorte que les points dans l'objet soient balayés une pluralité de fois ;

- une circuiterie de réception susceptible de recevoir les signaux électriques produits par les moyens transducteurs ultrasonores en réponse aux échos des dits signaux ultrasonores provenant de l'objet ;

- un générateur de signal d'écho susceptible de traiter les signaux électriques de la circuiterie de réception pour fournir des signaux d'écho sous une forme numérique adaptée à un traitement d'image ;

- une pluralité de moyens mémoire de sous- trames pour stocker les signaux d'écho dans une pluralité de plans mémoire en fonction de signaux d'adresse ;

- un processeur de lignes de flux (SLP), susceptible de traiter les données stockées dans les moyens mémoire de sous-trames pour obtenir les données représentant des segments de lignes de flux, comprenant :

    - un convertisseur de balayage numérique qui produit un signal d'écho d'entrée avec un signal d'adresse à utiliser pour le stockage dans les moyens mémoire de sous- trames ;

    - un processeur de signal numérique qui prend les différences entre les signaux d'écho concernant le même point dans l'objet pour obtenir les données représentant les images de taches ou points dans le flux et pour prendre les différences représentant le mouvement de taches dans le flux ;

    - une pluralité de commutateurs pour contrôler le flot de données et de signaux d'adresse entre le convertisseur de balayage numérique, le processeur de signal numérique et les moyens mémoire de sous- trames ;

    des moyens mémoire d'image pour stocker des données concernant des segments de lignes de flux ; et

    un circuit de visualisation fonctionnant sur la base des données stockées dans les moyens mémoire d'image et pour engendrer des signaux d'image pour représenter des segments de la ligne de flux sur un dispositif de visualisation à tube à rayons cathodiques CRT.

12. Dispositif selon la revendication 11, dans lequel le milieu est du sang et l'intervalle de temps ΔT entre les premier et dernier signaux d'écho concernant le même point dans l'objet, et entre lesquels on prélève une différence, est inférieur à D/V, où D est le diamètre moyen de la tache et V et la vitesse d'écoulement maximum envisagée.

13. Dispositif selon l'une quelconque des revendications 11 ou 12, dans lequel le processeur de traitement de signal (DSP) fonctionne de façon à comparer la valeur de la différence représentant le mouvement d'une tache dans le flux à une valeur de seuil, et de façon à quantifier la différence à un maximum prédéterminé ou une valeur minimum.

**Patentansprüche**

1. Ein Verfahren zum Erfassen einer Strömungslinie eines Flusses eines inhomogenen, innerhalb eines Objekts fließenden Mediums und zum Anzeigen einer Darstellung der Strömungslinie in Echtzeit, welches umfaßt:-

    (a) Aussenden von Ultraschallsignalen zu dem Objekt, in dem der Fluß vorkommt, um das Objekt entlang einer Vielzahl von Scanlinien zu scannen, wobei ein Scanbereich abgedeckt wird, so daß Punkte innerhalb des Objekts viele Male gescannt werden;

    (b) Erfassen von Echos der Ultraschallsignale von dem Objekt;

    (c) Erzeugen eines Bildes, welches die Unterschiede zwischen den von dem gleichen Punkt innerhalb des Objekts empfangenen Echos, die aber von zu verschiedenen Zeiten ausgesendeten Ultraschallsignalen stammen, darstellt, um dadurch die Bewegungskurve von Erscheinungen, die sich mit dem Fluß bewegen, als Darstellung der Strömungslinie zu erfassen und anzuzeigen.

2. Ein Verfahren nach Anspruch 1, bei dem die genannten Erscheinungen Flecken sind.

3. Ein Verfahren nach Anspruch 1, bei dem die genannten Erscheinungen in den Fluß eingeführte Blasen oder Partikel sind.

4. Ein Verfahren nach Anspruch 1, 2 oder 3, bei dem die Schritte (a) bis (c) bezüglich jedes von einer Vielzahl von verschiedenen, entsprechende Unterbereiche eines gesamten, zusammengesetzten Scanbereiches bildenden Scanbereichen durchgeführt werden.

5. Ein Verfahren nach irgendeinem vorhergehenden Anspruch, bei dem der oder jeder Schritt (a) mindestens die Vollendung zweier Scanrahmen des Scanbereiches umfaßt, und bei dem Schritt (c) das Aufnehmen von Unterschieden zwischen sich auf die gleiche Scanlinie beziehenden, aber von in verschiedenen Scanrahmen ausgesendeten Ultraschallsignalen stammenden Echos umfaßt.

6. Ein Verfahren nach irgendeinem vorhergehenden Anspruch, bei dem der oder jeder Schritt (a) das Scannen jeder Linie des Scanbereiches mindestens zweimal in einem Scanrahmen umfaßt, und bei dem Schritt (c) das Aufnehmen von Unterschieden zwischen sich auf verschiedene Scanoperationen derselben Scanlinie in demselben Scanrahmen beziehende Echos umfaßt.

7. Ein Verfahren nach Anspruch 2 oder irgendeinem der Ansprüche 4 bis 6 in Verbindung mit Anspruch 2, bei dem das Zeitintervall ΔT zwischen den ersten und letzten Zeiten, zu denen Ultraschallsignale ausgesendet werden, von denen Echos stammen, die zu der Schaffung des Abbildes des gleichen Punktes in dem Objekt beitragen, kleiner als D/V ist, wobei D der mittlere Fleckendurchmesser und V eine maximale (erwartete) Flußgeschwindigkeit ist.

8. Ein Verfahren nach irgendeinem vorhergehenden Anspruch, bei dem das Medium Blut ist und das Objekt das Herz ist.

9. Ein Verfahren nach Anspruch 8, bei dem die Schritte (a) bis (c) synchron mit den aufeinanderfolgenden Herzschlägen wiederholt werden.

10. Ein Verfahren nach irgendeinem vorhergehenden Anspruch, bei dem die Unterschiede mit einem Schwellenwert verglichen und auf ein vorher festgelegtes Maximum oder ein vorher festgelegtes Minimum quantisiert werden, je nachdem, ob sie den Schwellenwert überschreiten oder nicht.

11. Eine Anordnung zum Erfassen einer Strömungslinie eines Flusses eines inhomogenen, innerhalb eines Objekts fließenden Mediums, und zum Anzeigen einer Darstellung der Strömungslinie in Echtzeit, durch das Scannen des Objektes, in dem der Fluß vorkommt, mit Ultraschallsignalen und zum Verarbeiten der Echos jener Signale, wobei die Anordnung umfaßt:-
   ein Ultraschallwandlermittel, das betriebsfähig ist, die genannten Ultraschallsignale und ihre Echos auszusenden und zu empfangen;
   eine Übertragungsschaltung, die betriebsfähig ist, um das Wandlermittel zu erregen, um das Ultraschallwandlermittel zum Aussenden der genannten Ultraschallsignale zu veranlassen, so daß Punkte in dem Objekt viele Male gescannt werden;
   eine Empfangsschaltung, die betriebsfähig ist, um elektrische Signale, die durch das Ultraschallwandlermittel ansprechend auf Echos der genannten Ultraschallsignale von dem Objekt zur Verfügung gestellt werden, zu empfangen;
   einen Echosignalgenerator, der betriebsfähig ist, um elektrische Signale von der Empfangsschaltung zu verarbeiten, um Echosignale in digitaler Form, die für die Bildverarbeitung geeignet sind, zur Verfügung zu stellen;
   eine Vielzahl von Unterrahmen-Speichermitteln, die betriebsfähig sind, um Echosignale in einer Vielzahl von Speicherebenen in Übereinstimmung mit Adreßsignalen zu speichern;
   einen Strömungslinienprozessor (SLP), der betriebsfähig ist, um die in den Unterrahmen-Speichermitteln gespeicherten Daten zu verarbeiten, um Segmente von Strömungslinien darstellende Daten von Strömungslinien zu erhalten, der umfaßt:-
   einen digitalen Scankonverter, der ein Eingangsechosignal zum Zweck der Speicherung in den Unterrahmen-Speichermitteln mit einem Adreßsignal versieht;
   einen digitalen Signalprozessor, der die Unterschiede zwischen den sich auf denselben Punkt in dem Objekt beziehenden Echosignalen aufnimmt, um Daten zu erhalten, die Bilder von Flecken im Fluß darstellen, und der Unterschiede, die die Fleckenbewegung in dem Fluß darstellen, aufnimmt; und
   eine Vielzahl von Schaltern zum Steuern des Flusses der Daten und Adreßsignale zwischen dem digitalen Scankonverter, dem digitalen Signalprozessor und den Unterrahmen-Speichermitteln;
   ein Bildspeichermittel zum Speichern von sich auf Segmente von Strömungslinien beziehenden Daten; und
   eine auf der Basis von in dem Bildspeichermittel gespeicherten Daten betriebsfähige Anzeigeschaltung zum Erzeugen von Bildsignalen zum Darstellen von Segmenten der Strömungslinie auf einer Anzeige einer Katodenstrahlröhre (CRT).

12. Eine Anordnung nach Anspruch 11, bei der das Medium Blut ist und das Zeitintervall ΔT zwischen den ersten und letzten, sich auf denselben Punkt im Objekt beziehenden Echosignalen, und zwischen denen ein Unterschied aufgenommen wird, kleiner als D/V ist, wobei D der mittlere Fleckendurchmesser und V eine (erwartete) maximale Flußgeschwindigkeit ist.

13. Eine Anordnung nach irgendeinem der Ansprüche 11 oder 12, bei der der Digitalsignalprozessor (DSP) betriebsfähig ist, um den Wert eines die Fleckenbewegung in dem Fluß darstellenden Unterschiedes mit einem Schwellenwert zu vergleichen, und um den Unterschied auf einen vorher festgelegten maximalen oder minimalen Wert zu quantisieren.

(a)   (b)   (c)

(d)   (e)   (f)

FIG. 1

(a)

(b)

(1 div = 6.4 mS)

FIG. 2

FIG. 3

FIG. 4

*FIG. 5*

FIG. 6